Europäisches Patentamt

European Patent Office

Office européen des brevets

(11) Publication number: **0 330 706 B1**

# EUROPEAN PATENT SPECIFICATION

(45) Date of publication of patent specification: **10.06.92**  (51) Int. Cl.⁵: **C07H 17/08**, C12P 19/62,
//(C12P19/62,C12R1:465)

(21) Application number: **88102993.8**

(22) Date of filing: **29.02.88**

(54) **Method for forming amphotericin B crystals in fermentation broth.**

(43) Date of publication of application:
**06.09.89 Bulletin  89/36**

(45) Publication of the grant of the patent:
**10.06.92 Bulletin  92/24**

(84) Designated Contracting States:
**AT BE CH DE ES FR GB GR IT LI LU NL SE**

(56) References cited:
**FR-A- 2 173 632**
**US-A- 2 908 611**
**US-A- 4 177 265**

(73) Proprietor: **E.R. Squibb & Sons, Inc.**
**Lawrenceville-Princeton Road**
**Princeton, N.J. 08540-4000(US)**

(72) Inventor: **Ko, Raphael Y.**
**14 Marblehead Drive**
**Princeton Junction New Jersey(US)**
Inventor: **Szarka, Laszlo Joseph**
**5 Wellington Road**
**East Brunswick New Jersey(US)**

(74) Representative: **Vossius & Partner**
**Siebertstrasse 4 P.O. Box 86 07 67**
**W-8000 München 86(DE)**

## Description

The present invention relates to a method of fermentation especially adapted for use in high yielding amphotericin B fermentation and, more particularly, to a method for forming amphotericin B crystals directly in fermentation broths.

Until now, crystals of amphotericin B were prepared from fermentation broth through elaborate recovery procedures which included costly filtration, solvent extraction and crystallization steps.

In accordance with the present invention, crystallization of amphotericin B is induced directly in the fermentation broth which incorporates a water-soluble or water-insoluble fermentation medium, such as described in U. S. Patent No. 2,908,611, which fermentation medium includes a nitrogen source, a carbon/energy source, and optionally one or more inorganic salts for process control.

In addition, in accordance with the present invention, a process is provided for forming crystals of amphotericin B directly in fermentation broth which process includes providing a fermentation medium as described above, growing Streptomyces nodosus in such fermentation medium and inducing crystallization of amphotericin B and microbial cell autolysis. Crystallization and autolysis can be carried out by heating the broth at a sufficient temperature, at least 50°C and preferably from about 70°C to about 130°C, for a sufficient period, for example, from about 1 minute to about 10 hours, and preferably from about 10 to about 45 minutes, to induce formation of crystals of amphotericin B.

The crystallization can also be initiated by seeding amphotericin B crystals in the fermentation broth during the fermentation, preferably from about 10 to about 40 hours after start of fermentation.

Autolysis can also be carried out by the use of enzymes, such as lysozyme or other comparable enzyme which has lytic effect on the cell wall.

Where an enzyme or seeding is employed, less heat treatment may be necessary.

The fermentation media employed in the method will contain a nitrogen source in an amount within the range of from about 0.1 to about 20%, and preferably from about 0.5 to about 5% by weight of the media. Examples of suitable nitrogen sources include casein hydrolysate, cottonseed or its derivatives, corn steep liquor, soybean meal or any other comparable organic or inorganic N sources or their soluble derivatives.

The carbohydrate source will be present in the fermentation media in an amount within the range of from about 0.5 to about 20% and preferably from about 0.5 to about 10% by weight. Examples of suitable carbohydrate sources include starch, dextrin, sugars such as maltose, lactose and glucose, glycerol and the like.

The fermentation media employed in the method of the invention may optionally include other conventional fermentation medium components such as one or more inorganic salts which aid in process control. Examples of such inorganic salts include, but are not limited to, $CaCO_3$, $KH_2PO_4$, $(NH_4)_2SO_4$, $MgSO_4$, $MnSO_4$ or $Na_2HPO_4$. The fermentation media may also contain one or more antifoam agents such as silicone antifoam.

A preferred fermentation medium formulation includes from about 1 to about 6% by weight of organic nitrogen source, preferably soy bean meal, from about 2 to about 10% by weight glucose as the carbohydrate source, optionally from about 0.01% to about 0.2% by weight of one or more inorganic salts, preferably calcium carbonate and potassium dihydrogen phosphate, and optionally from about 0.05% to 2% by weight silicone antifoam.

In a preferred embodiment of the method of the invention for forming separable crystals of fermentation product (amphotericin B) in the fermentation broth, the fermentation medium may be completely water-soluble. The fermentation medium is used in the amphotericin fermentation employing the procedure as described in U. S. Patent No. 2,908,611.

The harvest broth is then heated at a temperature of at least 50°C for a sufficient length of time, for example, for 10-15 minutes to effect microbial cell autolysis which results in the formation of crystals of amphotericin B. In another embodiment, lysozyme or other proteolytic enzyme (such as proteases, glucanase, papain, trypsin or pepsin) may be added to the fermentation broth, at harvest, at a pH range of 5-7.5, and at a temperature of from about 20 to about 80°C for from about 30 minutes to about 5 hours. Then, the temperature is raised to 90°C or higher for 30 minutes to complete the crystal generation.

In another preferred method for carrying out the crystallization, during the fermentation from about 0.01 to about 0.1% w/v sterile amphotericin B crystals (based on volume of broth), can be introduced into the fermentor at 10 to 40 hours of fermentation; thereafter the fermentation is continued as described above, with heating (as described for the autolysis step) or without heating. At the end of the fermentation, amphotericin B microcrystals are present in the fermentation broth which crystals may be separated. The application of the heat is advantageous in addition to the seeding procedure.

The amphotericin crystals are easily separable from the broth by any solid/liquid separation procedure including a flotation technique.

The following Examples represent preferred embodiments of the present invention.

Example 1

Crystals of amphotericin B were prepared as described below.

A 20-liter batch of Streptomyces nodosus ATCC 14899 was fermented for the production of amphotericin B with the inoculum development and fermentation condition listed below:

Inoculum Development

A. First Stage

Inoculum Source: Frozen vial of culture Streptomyces nodosus ATCC 14899

Medium:

| Glucose | 1% |
|---|---|
| N-Z Amine B | 1.5% |
| Yeast Extract | 1.0% |
| NaCl | 0.5% |
| CaCO$_3$ | 0.1% |

pH adjusted to 6.8-7.0.
Volume: 100 ml in 500 ml flask.
Sterilization: 30 minutes at 121°C.
Temperature: 25°C.
Incubation: 48 hours on a reciprocating shaker at 220 rpm.

B. Second Stage

Inoculum Source: 10% from first stage.
Medium: Same as first stage.
Sterilization: 30 minutes at 121°C
Volume: 100 ml in 500 ml flask.
Temperature: 25°C
Incubation: 48 hours on a reciprocating shaker at 220 rpm.

Germination:

Medium:

| Nutrisoy Flour | 5.0% |
|---|---|
| Cornsteep Liquor | 1.6% |
| NaCl | 0.5% |
| Silicon Antifoam | 0.1% |

Sterilization: 40 minutes at 121°C.
Temperature: 28°C.
Agitation: 0.7 HP/400 l (100 U.S. Gallons).
Aeration: 1VVM (volume per volume per minute).
Germination Cycle: 28 hours.
Inoculum Size: 100 ml from the second flask stage.

Fermentation Condition:

Medium:

| Glucose | 7.0% |
|---|---|
| Soybean Oil Meal | 2.5% |
| CaCO$_3$ | 0.9% |
| KH$_2$PO$_4$ | 0.01% |
| Silicon Antifoam | 0.02% |

Sterilization: 45 minutes at 121°C.
Temperature: 25°C.
Agitation: 3.5 HP/400 l (100 U.S. gallons).
Aeration: 1 VVM.
Fermentation Cycle: 136 hours.
Inoculum Size: 14% from germination stage.
The resulting harvest broth contained 2050 $\gamma$/ml of amphotericin B and 390 $\gamma$/ml of amphotericin A.

Microscopic examination of the harvest broth did not reveal any crystals in the broth. The broth was heated at 60°C for 15 minutes. Upon microscopic examination, the broth was found to contain an abundance of crystals of amphotericin B which were easily separable from the harvest broth by flotation or other inexpensive separation techniques.

Example 2

Recovery of Amphotericin Powder from Fermentation Broth

The fermentation aspect of the Example was identical to Example 1 except the fermentation medium was filtered to remove undigested soybean residuals using a Sparkler filter lined with cotton fabric. The filtrate obtained was then sterilized again and used as fermentation medium. The resulting harvest broth contained 960 $\gamma$/ml of amphotericin B after 112 hours of fermentation. The fermentation broth was then cured at 120°C for several minutes to induce amphotericin crystal formation. After cooling to room temperature, the amphotericin crystals were then separated by centrifugation and dried at 15 mm Hg 25°C for 3 hours. Such recovered crystals were found to contain 79 $\gamma$ amphotericin B/mg of crystals with overall recovery yield of 95%.

Example 3

Enzymatic Treatment and Heating

Following the procedure in Example 2, after curing the fermentation broth at 120°C for several minutes to induce amphotericin B crystallization, the broth was cooled to and maintained at 35°C and pH around 7.0; then lysozyme was added to the broth and reacted for 2 hours to partially lyse the mycelial cells. Lysozyme was added at a concentration of 4000*unit/ml of broth. Amphotericin B crystals were then separated from the broth by centrifugation and acetone dried. Such powder was found to contain an even higher amphotericin B content than the product of Example 2.

Example 4

Seeding

The procedure of Example 2 was followed except that 0.1g/l of crystals of amphotericin B was added 40 hours after start of fermentation.

At the end of the heating step (after fermentation), crystals of amphotericin B were recovered.

*One unit is defined as the enzyme activity that will produce a $\Delta A_{450}$ of 0.001 per minute at pH 6.24 at 26°C.

## EP 0 330 706 B1

Example 5

Formation of Amphotericin B Crystals in Soluble Fermentation Medium

The fermentation aspect of this example was identical to Example 1 except 2.5% soybean oil in the fermentation medium was replaced with 1.25% soluble form of spray dried corn steep liquor (supplied by Roquette Corporation).

The broth was heated at about 75°C for 15 minutes. After the heat treatment the broth contained amphotericin B crystals and partially lysed mycelium. The crystals of amphotericin B were then separated from the liquid by centrifugation and they were dried at 15 mm Hg at 25°C for 3 hours. The overall recovery yield of amphotericin was 95%.

**Claims**

1. A method for producing crystals of amphotericin B directly in a fermentation medium, which consists essentially of cultivating a strain of Streptomyces nodosus in a fermentation medium which is substantially free of solvent for amphotericin B until substantial antifungal activity is imparted to said medium, heating the medium to a temperature of from about 70° to about 130°C for at least l0 minutes to form crystals of amphotericin B, and recovering substantial amounts of said crystals of amphotericin B from said medium.

2. The method for producing crystals of amphotericin B as defined in Claim 1 further including the step of adding crystals of amphotericin B to the fermentation medium to induce crystal formation.

3. The method as defined in Claim 1 wherein the medium containing the amphotericin B is heated for a period of from about 1 minute to about 10 hours to cause formation of crystals of amphotericin B.

4. The method as defined in Claim 3 wherein the fermentation broth containing the amphotericin B is heated to about 121°C for about 20 minutes to cause formation of crystals of amphotericin B.

5. The method for producing amphotericin B crystals as defined in Claim 1 further including the step of adding lysozyme, protease, glucanase, papain, trypsin or pepsin after the heating step to complete the crystal formation.

6. The method for producing amphotericin B crystals as defined in Claim 5 wherein lysozyme is added.

7. The method as defined in Claim 2 wherein crystals of amphotericin B are introduced into the fermentation medium from about 10 to about 40 hours after start of cultivating.

8. The method as defined in Claim 7 wherein said amphotericin B crystals are added in an amount of from about 0.01 to about 0.1% weight/volume based on the volume of fermentation medium.

9. The method as defined in Claim 1 wherein the fermentation medium is comprised of a nitrogen source in an amount within the range of from about 0.1 to about 20% by weight of the fermentation medium, a carbohydrate source in an amount within the range of from 0.5 to about 20% by weight of the fermentation medium, optionally one or more inorganic salts, and optionally one or more anti-foam agents.

10. The method as defined in Claim 9 wherein the nitrogen source is soybean oil meal or corn steep liquor, and the carbohydrate source is glucose.

11. The method as defined in Claim 9 including one or more inorganic salts to aid in process control.

12. The method as defined in Claim 10 wherein the inorganic salts are $CaCO_3$ and $KH_2PO_4$.

13. The method as defined in Claim 9 including a silicon antifoam agent.

**Revendications**

**1.** Procédé de production directe de cristaux d'amphotéricine B dans un milieu de fermentation, qui consiste essentiellement à cultiver une souche de Streptomyces nodosus dans un milieu de fermentation qui est pratiquement dépourvu de solvant de l'amphotéricine B jusqu'à ce qu'une action antifongique notable soit communiquée audit milieu, à chauffer le milieu à une température d'environ 70° à environ 130°C pendant au moins 10 minutes pour former des cristaux d'amphotéricine B, et à récupérer des quantités notables desdits cristaux d'amphotéricine B à partir dudit milieu.

**2.** Procédé de production de cristaux d'amphotéricine B selon la revendication 1, comportant en outre l'addition de cristaux d'amphotéricine B au milieu de fermentation pour induire la formation de cristaux.

**3.** Procédé selon la revendication 1, dans lequel on chauffe le milieu contenant l'amphotéricine B pendant un laps de temps d'environ 1 minute à environ 10 heures pour provoquer la formation de cristaux d'amphotéricine B.

**4.** Procédé selon la revendication 3, dans lequel on chauffe le bouillon de fermentation contenant l'amphotéricine B à une température d'environ 121°C pendant 20 minutes environ pour provoquer la formation de cristaux d'amphotéricine B.

**5.** Procédé de production de cristaux d'amphotéricine B selon la revendication 1, comportant en outre l'addition de lysozyme, de protéase, de glucanase, de papaïne, de trypsine ou de pepsine après l'étape de chauffage pour compléter la formation des cristaux.

**6.** Procédé de production de cristaux d'amphotéricine B selon la revendication 5, dans lequel on ajoute de la lysozyme.

**7.** Procédé selon la revendication 2, dans lequel on introduit des cristaux d'amphotéricine B dans le milieu de fermentation, d'environ 10 à environ 40 heures après le début de la culture.

**8.** Procédé selon la revendication 7, dans lequel on ajoute lesdits cristaux d'amphotéricine B en quantité d'environ 0,01 à environ 0,1 % en poids/volume, par rapport au volume du milieu de fermentation.

**9.** Procédé selon la revendication 1, dans lequel le milieu de fermentation se compose d'une source d'azote en quantité comprise dans l'intervalle d'environ 0,1 à environ 20 % du poids du milieu de fermentation, d'une source d'hydrate de carbone en quantité comprise dans l'intervalle d'environ 0,5 à environ 20 % du poids du milieu de fermentation, facultativement de un ou plusieurs sels minéraux, et facultativement de un ou plusieurs agents anti-mousse.

**10.** Procédé selon la revendication 9, dans lequel la source d'azote est la farine d'huile de soja ou la liqueur de macération de maïs, et la source d'hydrate de carbone est le glucose.

**11.** Procédé selon la revendication 9, comportant un ou plusieurs sels minéraux contribuant à la régulation du procédé.

**12.** Procédé selon la revendication 10, dans lequel les sels minéraux sont $CaCO_3$ et $KH_2PO_4$.

**13.** Procédé selon la revendication 9, comportant un agent anti-mousse à base de silicone.

**Patentansprüche**

**1.** Verfahren zur direkten Herstellung von Amphotericin B-Kristallen in einem Fermentationsmedium, wobei das Verfahren im wesentlichen besteht aus der Züchtung eines Stammes von Streptomyces nodosus in einem Fermentationsmedium, das im wesentlichen frei von einem Lösungsmittel für Amphotericin B ist, bis dem Medium eine wesentliche antifungale Aktivität verliehen worden ist, Erwärmen des Mediums auf eine Temperatur von etwa 70 bis etwa 130°C für eine Zeitspanne von mindestens 10 Minuten, um Amphotericin B-Kristalle zu bilden, und Gewinnen von wesentlichen Mengen an den Amphotericin B-Kristallen aus dem Medium.

6

**2.** Verfahren zur Herstellung von Amphortericin B-Kristallen gemäß Anspruch 1, das zusätzlich die Stufe der Zugabe von Amphotericin B-Kristallen zum Fermentationsmedium umfaßt, um die Kristallbildung zu induzieren.

**3.** Verfahren nach Anspruch 1, wobei das Medium mit einem Gehalt an Amphotericin B für eine Zeitspanne von etwa 1 Minute bis etwa 10 Stunden erwärmt wird, um die Bildung von Amphotericin B-Kristallen hervorzurufen.

**4.** Verfahren nach Anspruch 3, wobei die Fermentationsbrühe mit einem Gehalt an Amphotericin B etwa 20 Minuten auf etwa 121°C erwärmt wird, um die Bildung von Amphotericin B-Kristallen hervorzurufen.

**5.** Verfahren zur Herstellung von Amphotericin B-Kristallen gemäß Anspruch 1, das zusätzlich die Stufe der Zugabe von Lysozym, Protease, Glucanase, papain, Trypsin oder Pepsin nach der Erwärmungsstufe umfaßt, um die Kristallbildung zu vervollständigen.

**6.** Verfahren zur Herstellung von Amphotericin B-Kristallen gemäß Anspruch 5, wobei Lysozym zugegeben wird.

**7.** Verfahren gemäß Anspruch 2, wobei Amphotericin B-Kristalle in das Fermentationsmedium für etwa 10 bis etwa 40 Stunden nach Kultivierungsbeginn eingebracht werden.

**8.** Verfahren gemäß Anspruch 7, wobei die Amphotericin B-Kristalle in einer Menge von etwa 0,01 bis etwa 0,1 Gewichtsprozent/Volumen, bezogen auf das Volumen des Fermentationsmediums, zugegeben werden.

**9.** Verfahren gemäß Anspruch 1, wobei das Fermentationsmedium aus einer Stickstoffquelle in einer Menge im Bereich von etwa 0,1 bis etwa 20 Gewichtsprozent des Fermentationsmediums, einer Kohlenhydratquelle in einer Menge im Bereich von 0,5 bis etwa 20 Gewichtsprozent des Fermentationsmediums, gegebenenfalls einem oder mehreren organischen Salzen, und gegebenenfalls einem oder mehreren Antischaummitteln besteht.

**10.** Verfahren gemäß Anspruch 9, wobei die Stickstoffquelle Sojaextraktionsschrot oder Maisquellflüssigkeit und die Kohlenhydratquelle Glucose ist.

**11.** Verfahren gemäß Anspruch 9, umfassend ein oder mehrere anorganische Salze zur Unterstützung bei der Verfahrenssteuerung.

**12.** Verfahren gemäß Anspruch 10, wobei die anorganischen Salze $CaCO_3$ und $KH_2PO_4$ sind.

**13.** Verfahren gemäß Anspruch 9, umfassend ein Silizium-Antischaummittel.